# EUROPEAN PATENT SPECIFICATION

(11) **EP 0 708 828 B1**
(45) Date of publication and mention of the grant of the patent: **12.03.1997**
(21) Application number: 94920537.1
(22) Date of filing: 08.07.1994
(51) Int. Cl.: C12N 15/10, C12P 19/34

(54) **OBTAINING NUCLEIC ACIDS**
HERSTELLUNG VON NUKLEINSÄUREN
PROCEDE POUR OBTENIR DES ACIDES NUCLEIQUES

(30) Priority: 08.07.1993 GB 9314175; 08.07.1993 GB 9314119
(43) Date of publication of application: 01.05.1996
(73) Proprietor: TEPNEL MEDICAL LIMITED, Knutsford, Cheshire WA16 9PD (GB)
(72) Inventor: MINTER, Stephen, New Mills SK12 4QL (GB)
(74) Representative: Atkinson, Peter Birch
(86) International application number: GB9401492
(87) International publication number: WO9502050

(56) References cited:
- EP-A- 0 574 267
- WO-A-92/11864
- US-A- 4 908 318
- THE MALAYSIAN JOURNAL OF PATHOLOGY, vol.12, no.1, June 1990, pages 39-41; LIM L. H. ET AL: 'A comparative study of two methods for the isolation of human leucocytes for DNA extraxction'
- METHODS IN ENZYMOLOGY, vol.108, 1984, pages 88-101; BOYUM A.: 'Separation of lymphocytes, granulocytes, and monocytes from human blood using iodinated density gradient media'
- CRITICAL CARE NURSE, vol.3, no.4, 1983, pages 38-40; SJOGREN E. R.: 'HESPAN (Hetastarch)'
- THE MEDICAL LETTER, vol.23, no.4, 1981, page 16; AUTHOR NOT AVAILABLE: 'Hetastarch (HESPAN) -A new plasma expander'

## Description

The present invention relates to a method of obtaining a nucleic acid from a sample of blood.

Various procedures require the isolation of nucleic acids (DNA or RNA) from blood. For example the nucleic acid may be required for sequencing of a gene present therein either for the purpose of research or clinical diagnosis. Alternatively the nucleic acid may be required for amplification thereof to obtain larger quantities for analysis purposes.

According to the present invention there is provided a method of obtaining a nucleic acid from a sample of blood comprising the steps of
treating the blood in vitro with a high molecular weight carbohydrate having negative charges along the carbohydrate backbone,
allowing the red blood cells to sediment, and obtaining a sample of the supernatant liquid,
lysing cells in said sample of the supernatant with a lysis mixture containing a surface active agent and a protein digesting enzyme.

The invention is most preferably used for obtaining DNA but may also be used for obtaining RNA.

The liberated nucleic acid of interest may be finally isolated by hybridisation to a suitable probe, e.g. using the column technique disclosed in our earlier PCT Application No. PCT/GB 92/02394 (WO-A-93/13220).

The nucleic acid to be isolated may for example be one which is known to be present in the sample. Alternatively the method may be effected to establish whether or not a particular nucleic acid sequence is present in the sample. Thus, for example, it may be desired to test whether the sample contains a particular virus or bacteria (characterised by a certain nucleic acid sequence). In this case, the incubated lysis mixture (or a purified extract thereof) may be probed with a hybridisation probe which is specific for the sequence of interest. Known techniques may be used to determine whether hybridisation has occurred and thus to confirm the presence or absence of the sequence in the original sample.

For preference the blood is anti-coagulated blood. The anticoagulating agent may for example be heparin or EDTA.

In the first step of the process, separation of red cells from the blood is effected by treatment of whole blood with a high molecular weight carbohydrate (e.g.10,000-1,000,000 Daltons) having negative charges along the carbohydrate backbone. We have found that high molecular weight carbohydrates with anionic charges on the backbone when added to whole blood produce a relatively rapid sedimentation of red cells which (when separated) leave a clear suspension of white cells and plasma from which DNA (or other nucleic acid) may be isolated.

The carbohydrate may be added to the blood at room or slightly elevated temperature. Sedimentation of red cells, will occur in a relatively short period of time (e.g. 10-15 minutes at Room Temperature) after which the supernatant plasma may readily be separated from the aggregated red cells. Provided that the sedimentation is not continued for too long the white cells will remain in the supernatant plasma and nucleic acid extracted from the white cells during the subsequent lysis procedure. If however the presence of white cells in the separated plasma is not required or desired then the sedimentation may be allowed to continue so that the white cells are also precipitated.

The carbohydrate may for example be dextran sulphate or more preferably one composed of (or substantially of) amylopectin into which negative charges have been introduced. The preferred carbohydrate is Hetastarch which is derived from a waxy starch composed almost entirely of amylopectin. In Hetastarch, hydroxyethyl ether groups have been introduced into the glucose unit of the starch and the resultant compound hydrolysed. The glucoses are primarily 1-4 linked and the hydroxyethyl ether group is introduced at the 2-position on the ring. The degree of substitution is preferably less than 2%, e.g. less than 1%. For example the degree of substitution may be 0.7% (7 hydroxyethyl ether groups for every 10 glucoses).

The Hetastarch may be used as a solution containing up to 10% of the active ingredient.

A suitable form of Hetastarch for use in the invention is a "blood bulker" available from Du Pont under the name Hespan which is supplied as a clear to amber liquid comprising 6% hetastarch (molecular weight ca 450,000 Daltons) and 0.9% sodium chloride. The osmolarity is 310m Osm/litre (calculated).

The amount of carbohydrate added to the blood is not critical although typically will be in less than 0.1 g/ml of blood although the lower the amount the longer will be the sedimentation time. We have obtained particularly good results using about 0.5 ml of Hespan per ml of whole blood.

The protein digesting enzyme used in the lysis step is preferably proteinase K although other non-specific proteases may be used.

The amount of the protein digesting enzyme used in the lysis is not believed to be critical although will generally be in the range 1-5 µg/µl (1-5 mg/ml). The lower amount of protein digesting enzyme used then the higher lysis temperature and/or longer lysis times may be required.

The surface active agent used in the lysis mixture will generally be either an anionic or non-ionic surfactant, the former being preferred. It is believed that the anionic surfactants de-stabilise inter-protein linkages by neutralising charges on protein molecules which are thus broken down into fragments and therefore more readily digested by the enzyme (e.g. proteinase K).

Particularly preferred anionic surface active agents are C₈₋₂₂ alkyl sulphates, particularly the alkali metal sulphates. The most preferred surface active agent is sodium dodecyl sulphate.

The amount of surface active agent used will generally be less than 5% more preferably less than 2%.

The lysis mixture will also include a suitable buffer to maintain the required pH, e.g. 6-8.

If it is desired to obtain RNA as the nucleic acid then the lysis mixture may need to include an agent (e.g. EDTA) to bind or chelate RNAse cofactor (e.g. magnesium) to prevent RNA being degraded during lysis.

The (i) time and (ii) temperature required for the incubation with the lysis mixture will depend on the amount of protein digesting enzyme used but will typically be (i) less than 1 hour and (ii) from room temperature to 50°C.

If an anionic surface active agent is used in the lysis mixture then it will generally be found that the nucleic acid may be "recovered" by introducing the mixture onto a column of solid particles having bonded thereto oligonucleotide probes which will hybridise to the nucleic acid of interest in the mixture (i.e. using the techniques described in WO-A-93/13220

If however a non-ionic surface active agent is used in the lysis mixture it may be necessary for the incubated mixture to be purified (e.g. using phenol/chloroform) before introduction onto the column. This is because the incubated mixture may still include large protein fragments which need to be removed before the mixture is introduced onto the column.

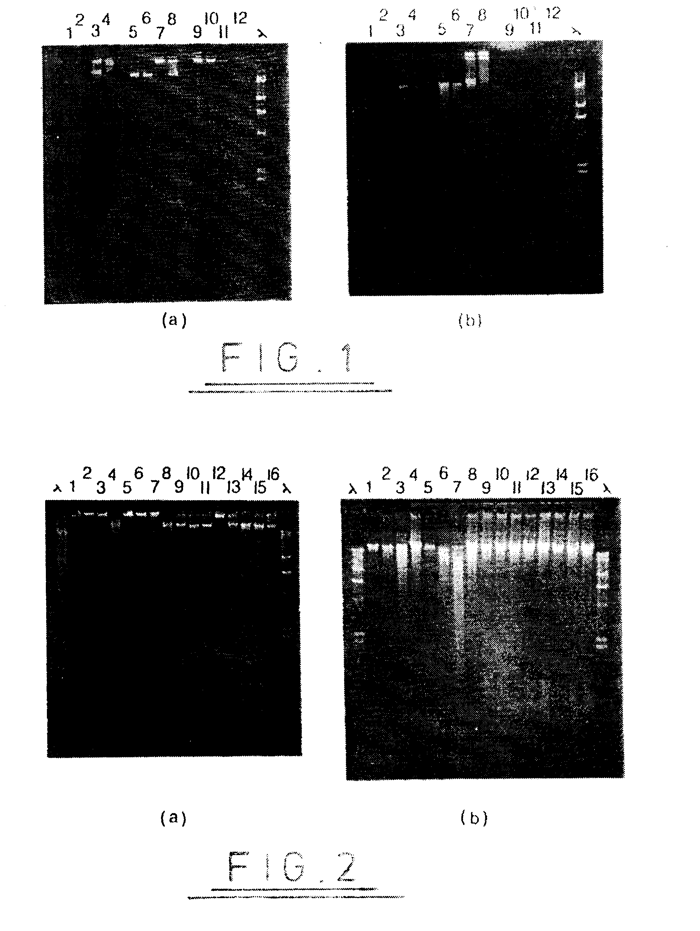

The invention will be further described by way of example only with reference to the following non-limiting Procedures and Examples in conjunction with Figs. 1-4 of the accompanying drawings.

In outline, the Examples described below outline the liberation of DNA (or RNA) from whole blood by the use of various lysis mixtures and boiling in order to identify techniques which would produce DNA (or RNA) suitable for application to, and manipulation on, a column as disclosed in WO-A-93/13220. In the context of the Examples, the suitable techniques are those which produce a DNA (or RNA) which is sufficiently free of protein to allow it to run into an electrophoresis gel and produce a single band without significant shearing.

As indicated above, the Examples illustrate liberation of DNA from whole blood and blood which has been treated by Hespan. Such treatment is described in Procedure 1 below. Lysis of the samples was effected using the technique and materials described in Procedure 2.

The products obtained were compared qualitatively by immediate gel electrophoresis of 20 µl aliquots of each crude preparation (Procedure 3). The preparations were then purified by phenol extraction, as described in Procedure 4. The purification process was begun as soon as possible after completion of the lysis incubation, to remove the lytic reagents and prevent release of further DNA, and was completed the following day. The DNA was then precipitated by salt and ethanol and resuspended in 50 µl TE, as described in Procedure 5.

20 µl aliquots of each purified DNA preparation were run on an agarose gel as before, to assess the DNA quality.

### Procedure 1

### Sedimentation of red blood cells by treatment with Hespan Materials

### Hespan = 6% hetastarch, supplied by DuPont Pharmaceuticals Ltd, product licence no. PL 11173/0015.

### Method

One part Hespan was added to four parts heparinised blood in a universal container. After mixing by gentle inversion, the tube was allowed to stand at 37°C or at room temperature and the sedimentation monitored.

After 10-15 min the majority of red blood cells had settled and typically 2 mls of straw coloured supernatant with a faint haze of red blood cells could be obtained from 5 mls of blood. Sedimentation was not allowed to continue further as the white blood cells might also eventually settle out.

The volume of the supernatant was restored to that of the original blood sample by the addition of sterile isotonic saline.

### Procedure 2

### Methods of cell lysis for the release of DNA

Three different lysis mixtures were used, all being evaluated alone and with proteinase K at 100 to 300 µg/ml. The reactions were set up as detailed in Table 1 and incubated for periods of 15 min to 1 hr at temperatures ranging from room temperature to 65°C.

### Reagents

### 1M Tris/HCl buffer pH 7.0 and 8.0.

121.14 g Tris (Hydroxyethyl) methylamine (Fisons, AR grade, ref T3712/60) was dissolved in 500 ml ultrapure water. The pH was adjusted with concentrated HCl and the solution made up to 1 litre with water. The solutions were aliquotted into 100 mls, autoclaved and the pH checked. The pH 8.0 buffer was subsequently further aliquotted into 5 ml portions, re-autoclaved and the pH of one aliquot checked again.

### 3M sodium acetate buffer pH 5.2

40.8 g sodium acetate 3H₂O (BDH, AR grade, ref 10235) was covered with ultrapure water to 75 ml and dissolved on a stirrer. The pH was brought to 5.2 with glacial acetic acid and the solution diluted to 100 ml with ultrapure water. It was split into 5 ml aliquots, autoclaved and the pH of one aliquot checked.

### 10% SDS

100 g sodium dodecyl sulphate (Fisons PrimaR reagent S/5202) was left to stand in 800 ml ultrapure water. Once dissolved the solution was made up to 1 litre with ultrapure water.

### 0.5M EDTA

186.12 g EDTA (BDH, AR grade, ref 10093) was dissolved in 500 ml ultrapure water on a stirrer in the fume cupboard. 5M NaOH was added dropwise and the pH allowed to stabilise between additions until the solution cleared, producing a final pH of approximately 8.0. The solution was diluted to 1 litre, filtered into 100 ml bottles and autoclaved.

### 0.1M Tris HCl, pH 7,0, 100 mM EDTA, 10% Triton-X-100

The following solutions were combined into a sterile bottle:

| | |
|---|---|
| 1M Tris HCl pH 7.0 | 10 mls |
| 0.5M EDTA | 20 mls |
| Triton-X-100 (BDH, 30632, GPR) | 10 mls |
| Sterile ultrapure water | 60 mls |

### Proteinase K

Supplied by Boehringer Mannheim GmbH, ref no 1000 144, lot 1319 5820-33, expiry date April 1993.

A solution of 10 mg/ml in sterile ultrapure water was prepared and stored as 50 µl at -20°C.

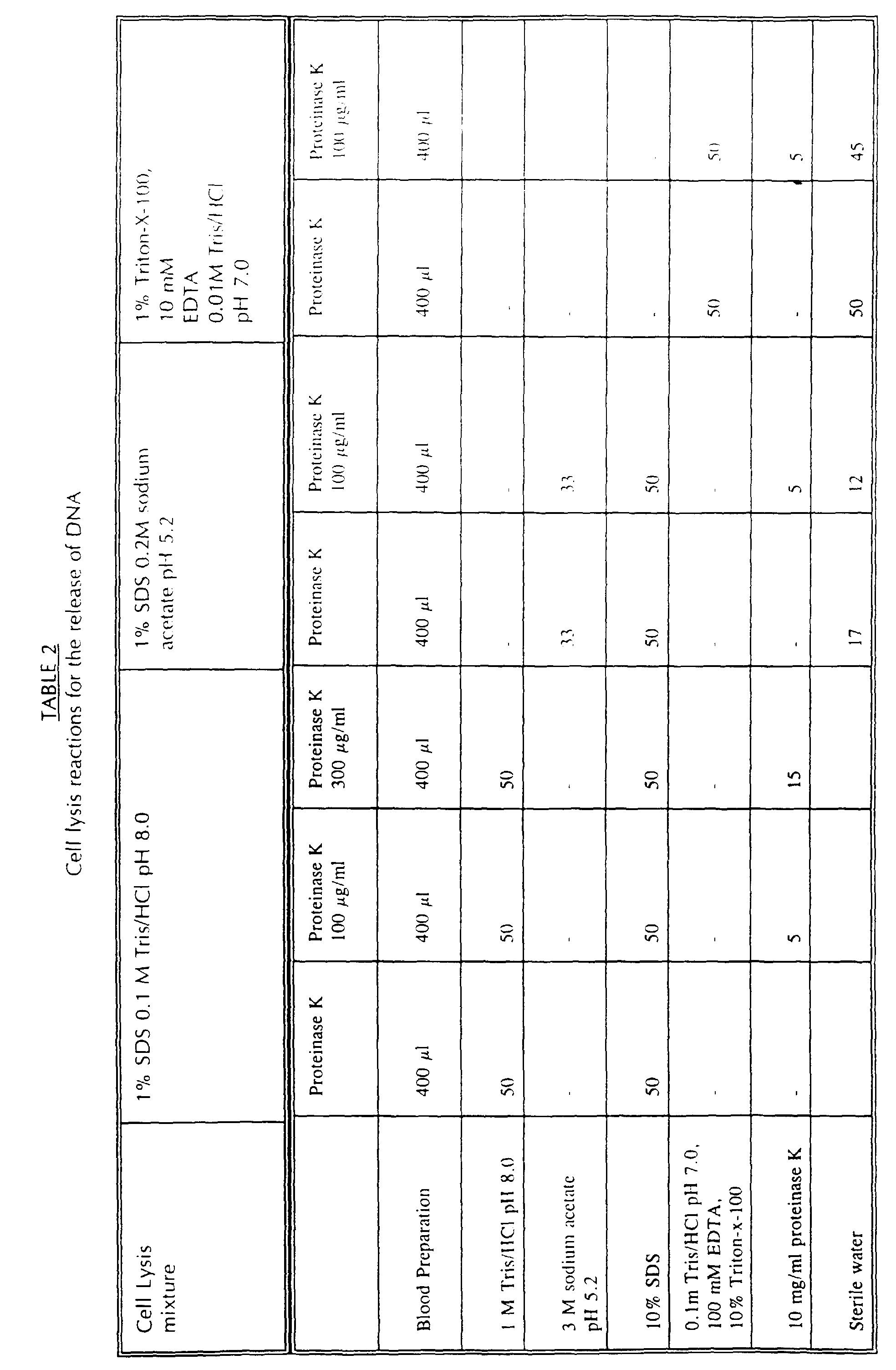

### Procedure 3

### Electrophoresis of DNA samples

20 µl aliquots of the DNA preparations were run in a submerged 12 cm + 12 cm gel of 0.8% agarose (Seakem HMT) in 1 x TAE (40 mM Tris acetate, 1 mM EDTA). 1 µg Hind III cut DNA was run alongside as a size marker.

The preparations were not pre-incubated at 65°C before loading the gel as this may have further assisted lysis and confused the comparison of lysis methods.

The gels containing the crude preparations were run overnight at 20V, while the gels containing the purified preparations were run for 5 hours at 60V, giving a similar separation. They were stained for 45 min in ethidium bromide (0.5 µg/ml in 1xTAE) and photographed under UV illumination.

### Procedure 4

### Purification of DNA preparations by phenol extraction

### Reagents

1. Phenol/chloroform/isoamyl alcohol (Phe/CHCl₃/IAA)
   Phenol (washed with Tris buffer,Fisons BioScience gradeP/2318),
      25 parts
   Chloroform (BDH AR grade, ref 10077),
      24 parts
   Isoamyl alcohol (BDH AR grade, ref 10038),
      1 part
   were mixed in the above proportions and stored at 4°C under 40 mM Tris/HCl pH 8.0 to maintain an alkaline pH.
2. Chloroform/isoamyl alcohol (CHCl₃/IAA)
   Prepared as above, omitting phenol and Tris buffer.

### Method

An equal volume of appropriate buffer, according to lysis method, was added to each preparation to reduce the viscosity and thereby facilitate removal of the aqueous phase. The diluted preparations were divided into two, and one 500 µl aliquot was retained to back-extract the organic phases.

Equal volumes of Phe/CHCl₃/IAA were added to each DNA preparation and mixed gently by inversion. The phases were separated by micro-centrifugation for 1 min, a speed of 13,000 rpm being needed to consolidate any material at the interface. The upper aqueous phases were removed and extracted twice more with Phe/CHCl₃/IAA, spinning as before. Further dilution of some samples, according to viscosity, was needed to permit clean removal of aqueous phases. This was followed by two extractions with CHCl₃/IAA alone to remove phenol.

The retained 500 µl aliquots of each preparation were then used to back extract the series of organic phases from each preparation. The resulting primary and back extracts from each preparation were pooled.

### Procedure 5

### Precipitation and resuspension of DNA

DNA was precipitated by the addition of 1M NaCl to a final concentration of 0.1M, followed by two volumes of filtered absolute ethanol at -20°C. After mixing by inversion, the tubes were chilled at -70°C for 30 min and left overnight at -20°C.

Precipitated DNA was pelleted by spinning in a microfuge at 13,000 rpm for 10 min at 4°C and the supernatant decanted. The pellets were washed in 1 ml 80% ethanol at -20°C and spun as before. The supernatant were again decanted and the necks of the tubes wiped with a tissue. The tubes were then left inverted on a tissue to drain for about 15 min until only traces of moisture remained.

A total of 50 µl 1 x TE, pH 8.0 (10 mM Tris/HCl, 1 mM EDTA) was added to each preparation and left overnight at 4°C to resuspend. A further incubation of approximately 1 hr at 65°C was needed the following day to complete the resuspension.

### Example 1

This Example illustrates the use of various lysis techniques for the treatment of whole blood and Hespan treated samples of blood. The results obtained by gel electrophoresis are shown in Figs. 1(a) and (b), the former illustrating the results for unpurified preparations and the latter for preparations purified in accordance with Procedure 3.

The lysis techniques used are summarised below in conjunction with the lane number on the gel showing the results of that technique. Note that identical lane numbers in Figs 1(a) and (b) relate to the same lysis technique and differ only in that the lane of 1(b) is in respect of the purified preparation.

| Lane No. | Blood Pretreatment | Lysis technique |
|---|---|---|
| 1 | None | 1% SDS in 0.2M sodium acetate pH 5.2, with proteinase K at 100 µg/ml.* |
| 5 | Hespan at 37°C | |
| | | |
| 2 | None | 1% SDS in 0.2M sodium acetate pH 5.2, without proteinase K.* |
| 6 | Hespan at 37°C | |
| | | |
| 3 | None | 1% SDS in 0.1M Tris pH 8.0, with proteinase K at 100 µg/ml.* |
| 7 | Hespan at 37°C | |
| | | |
| 4 | None | 1% SDS in 0.1M Tris pH 8.0, without proteinase K.* |
| 8 | Hespan at 37°C | |
| | | |
| 9-12 | Hespan at 37°C | Boiling for 1 min, 2 min, 5 min and 10 min. |

| | | |
|---|---|---|
| * Lysis effected by incubation at 37°C for 1 hr. | | |

### Results

### (a) 1% SDS in 0.1M Tris pH 8.0 (Lanes 3,4,7 and 8),

Using this lysis technique DNA was liberated from untreated blood (i.e. whole blood) and Hespan treated blood but the amount in the whole blood preparations appeared to decrease after purification. However the results of Example 2 (see infra) suggest that while Hespan treated blood produced DNA under these conditions, whole blood needed incubation at higher temperature. In this Example lysis may have been assisted by the fact that the preparations were left for 2 hrs and the aliquots heated to 65°C for 10 minutes before running in initial gels.

### (b) 1% SDS in 0.2M sodium acetate pH 5.2 (Lanes 1, 2, 5 and 6)

Much precipitate was produced in the whole blood preparations with considerably less in the Hespan treated preparations, and DNA was only detected in the Hespan treated blood (lanes 5 and 6). The presence of proteinase K in the lysis mixture seemed to make little difference to the amount of DNA liberated.

### (c) DNA Release by Boiling

This caused whole blood to coagulate and these samples were therefore discarded. Small amounts of DNA from the Hespan treated blood were detected on the gel of unpurified preparations but it did not migrate out of the loading well (Fig. 1a, lanes 9-10) and no DNA was detectable after purification.

### Example 2

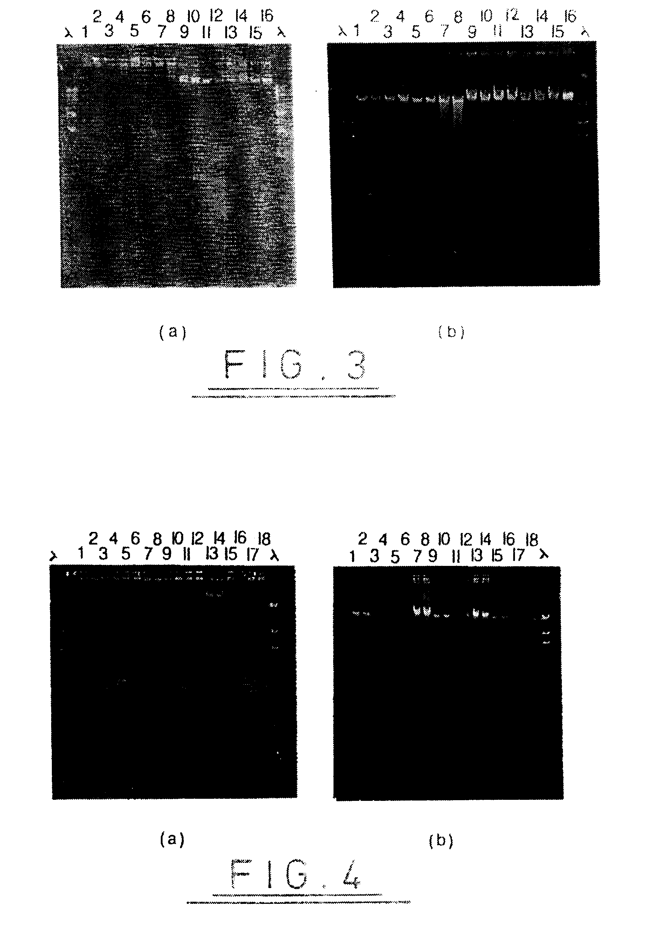

This Example investigates the use of a lysis mixture comprising 1% SDS in 0.1M Tris pH 8.0 incorporating various amounts of proteinase K and various incubation temperatures (using a fixed lysis time of 1 hour). The results are shown in Figs. 2(a) and (b) in which the former figure illustrates results for unpurified preparations and the latter for preparations purified in accordance with Procedure 3.

The lysis techniques are summarised below in conjunction with the lane number on the gel showing the results of that technique.

| Lane No. | Blood Pretreatment | Amount of Proteinase K | Incubation Temp. |
|---|---|---|---|
| 1 | None | 100 µg/ml | Room Temp |
| 2 | None | 100 µg/ml | 37°C |
| 3 | None | 100 µg/ml | 50°C |
| 4 | None | 100 µg/ml | 65°C |
| 5 | None | 300 µg/ml | Room Temp |
| 6 | None | 300 µg/ml | 37°C |
| 7 | None | 300 µg/ml | 50°C |
| 8 | None | 300 µg/ml | 65°C |
| 9 | Hespan at 37°C | 100 µg/ml | Room Temp |
| 10 | Hespan at 37°C | 100 µg/ml | 37°C |
| 11 | Hespan at 37°C | 100 µg/ml | 50°C |
| 12 | Hespan at 37°C | 100 µg/ml | 65°C |
| 13 | Hespan at 37°C | 300 µg/ml | Room Temp |
| 14 | Hespan at 37°C | 300 µg/ml | 37°C |
| 15 | Hespan at 37°C | 300 µg/ml | 50°C |
| 16 | Hespan at 37°C | 300 µg/ml | 65°C |

The lysis time was 1 hr in each case.

### Results

The gels run on the crude preparations suggested that the whole blood (Fig 2a lanes 1-8) needed incubation at 65°C with either concentration of proteinase K before satisfactory DNA was produced, while Hespan treated blood yielded suitable DNA with proteinase K at 100 µg/ml (Fig. 2a lane 9). The gel run of the purified preparations (Fig. 2b) showed free DNA in all samples but this was probably due to the unavoidable delay before purification permitting further lysis. The whole blood preparations showed increasing amounts of DNA with increasing temperature but higher temperature caused some shearing of DNA. The Hespan treated preparations all produced identical amounts of DNA regardless of lysis temperature.

Lanes 9-16 of Fig. 2 show that significant amounts of degraded RNA were produced from the Hespan treated blood samples.

### Example 3

This Example investigates the use of a lysis mixture comprising 1% SDS in O.1M Tris pH 8.0 with addition of various amounts of proteinase K using various incubation times and temperatures.

The results are shown in Figs. 3(a) and (b) in which the former figure illustrates results for unpurified preparations and the latter for preparations purified in accordance with Procedure 3.

The lysis techniques are summarised below in conjunction with the lane number on the gel showing the results of that technique.

| Lane Time | Blood Pretreatment | Amount of Proteinase K | Incubation Temp | Incubation |
|---|---|---|---|---|
| 1 | None | 100 µg/ml | Room Temp | 30 mins |
| 2 | None | 100 µg/ml | Room Temp | 60 mins |
| 3 | None | 100 µg/ml | 50°C | 15 mins |
| 4 | None | 100 µg/ml | 50°C | 30 mins |
| 5 | None | 300 µg/ml | Room Temp | 30 mins |
| 6 | None | 300 µg/ml | Room Temp | 60 mins |
| 7 | None | 300 µg/ml | 50°C | 15 mins |
| 8 | None | 300 µg/ml | 50°C | 30 mins |
| 9 | Hespan at 37°C | 100 µg/ml | Room Temp | 30 mins |
| 10 | Hespan at 37°C | 100 µg/ml | Room Temp | 60 mins |
| 11 | Hespan at 37°C | 100 µg/ml | 50°C | 15 mins |
| 12 | Hespan at 37°C | 100 µg/ml | 50°C | 30 mins |
| 13 | Hespan at 37°C | 300 µg/ml | Room Temp | 30 mins |
| 14 | Hespan at 37°C | 300 µg/ml | Room Temp | 60 mins |
| 15 | Hespan at 37°C | 300 µg/ml | 50°C | 15 mins |
| 16 | Hespan at 37°C | 300 µg/ml | 50°C | 30 mins |

### Results

No DNA capable of migration into the gel was detected in the whole blood sample after incubation for 30 mins or 60 mins at room temperature, or for 15 mins or 30 mins at 50°C (Fig. 3a, lanes 1-8?)

However, after purification, all treatments appeared to have produced DNA although it was considerably sheared after 15 mins or 30 mins incubation at 50°C with proteinase K at 300 µg/ml (Fig. 3b, lanes 7 and 8). Again, this difference between the gels could be due to the unavoidable delay before purification permitting further lysis. All treatments, even incubation at room temperature of 30 minutes with proteinase K at 100 µg/ml, produced satisfactory DNA from the Hespan treated samples.

### Example 4

This Example investigates the use of various lysis mixtures, lysis times, and amount of proteinase K. In all cases, the lysis was conducted at Room Temperature.

The results are shown in Figs. 4(a) and 4(b) in which the former figure illustrates results for unpurified preparations and the latter for preparations purified in accordance with Procedure 3.

The lysis techniques are summarised below in conjunction with the lane number on the gel showing the results of that technique.

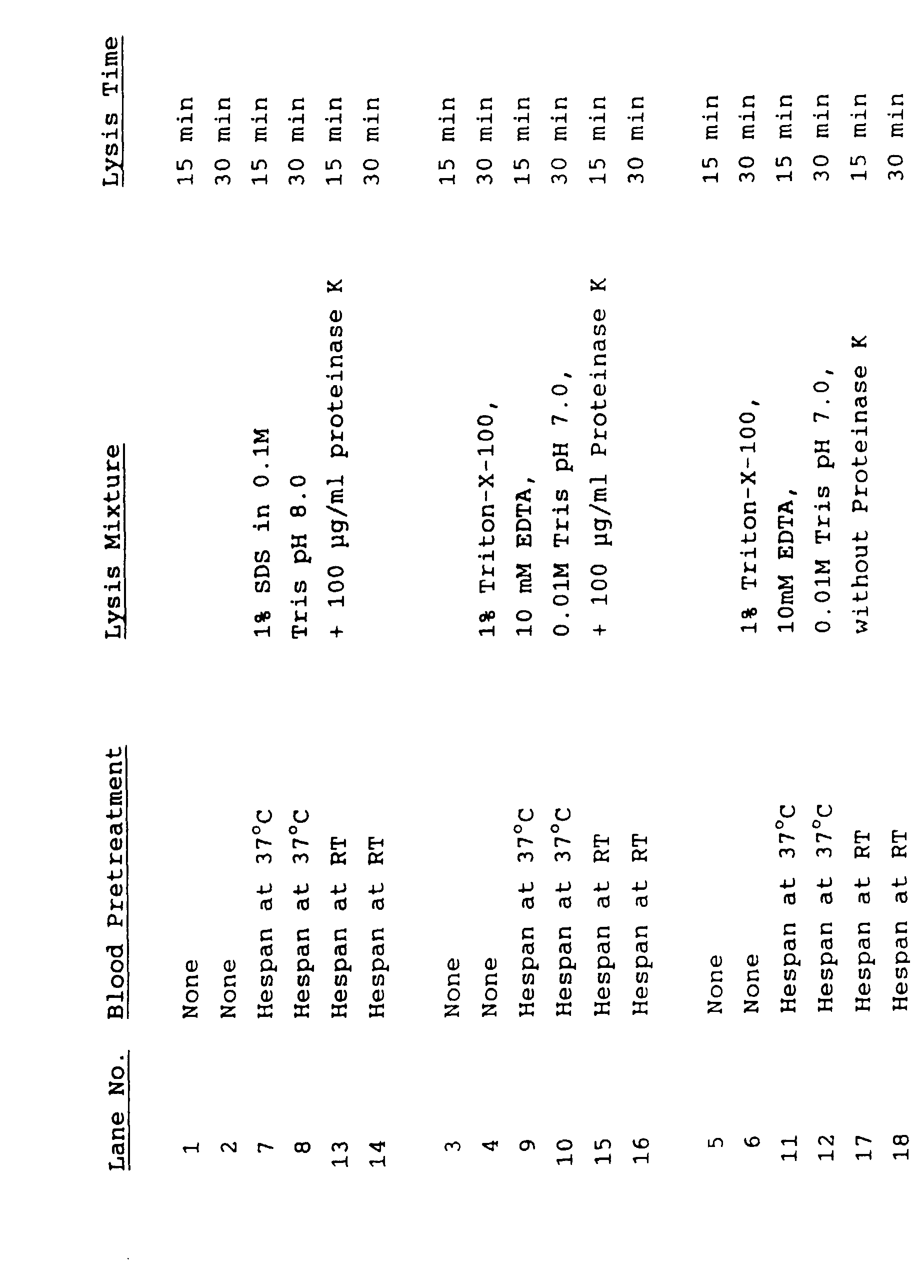

### Results

Lane 7 of Figs 4(a) and 4(b) demonstrates that a reduction in lysis incubation time to 15 minutes at room temperature again produced satisfactory DNA from Hespan treated blood samples which were lysed with a proteinase K/SDS/Tris pH 8.0 lysis mixture.

So far as use of a lysis mixture containing Triton-X-100 is concerned (lanes 3-6, 9-12, and 16-18), the gels from the crude preparations indicated no detectable DNA was released by this method, but after purification (and therefore an inevitably longer effective lysis incubation), small amounts of DNA could be detected from the Hespan treated blood when proteinase K was included in the lysis mixture (Fig. 4b, lanes 9-10, 15-16).

Furthermore significant amounts of intact RNA (migrating with the 4kb size marker) were obtained from the Hespan treated blood lysed with a mixture containing Triton-X-100 (Fig. 4a, lanes 3-6, 9-12, 15-18). It was not clear from the gels whether the presence of proteinase K was necessary for this.

## Claims

1. A method of obtaining a nucleic acid from a sample of blood comprising the steps of
treating the blood in vitro with a high molecular weight carbohydrate having negative charges along the carbohydrate backbone,
allowing the red blood cells to sediment, and obtaining a sample of the supernatant liquid,
lysing cells in said sample of the supernatant with a lysis mixture containing a surface active agent and a protein digesting enzyme.

2. A method as claimed in Claim 1 wherein the blood is anti-coagulated blood.

3. A method as claimed in claim 1 or 2 wherein the amount of carbohydrate added to the blood is less than 0.1 g per ml of blood.

4. A method as claimed in any one of Claims 1 to 3 wherein the carbohydrate has a molecular weight of 10,000-1,000,000 Daltons.

5. A method as claimed in any one of Claims 1 to 4 wherein the carbohydrate is one composed of (or substantially of) amylopectin into which negative charges have been introduced.

6. A method as claimed in Claim 5 wherein the negative charges are provided by hydrolysis of hydroxyethyl ether groups which have been introduced into glucose units of the amylopectin.

7. A method as claimed in Claim 6 wherein the degree of substitution of the hydroxyethyl ether groups is less than 2%.

8. A method as claimed in any one of Claims 1 to 7 wherein the treatment of the blood with the high molecular weight carbohydrate is effected for a period of time such that white cells remain in the supernatant plasma.

9. A method as claimed in any one of Claims 1 to 8 wherein the protein digesting enzyme used in the lysis step is proteinase K.

10. A method as claimed in any one of Claims 1 to 9 wherein the amount of the protein digesting enzyme used in the lysis is 1-5 mg/ml.

11. A method as claimed in any one of Claims 1 to 10 wherein the surface active agent is an anionic surfactant.

12. A method as claimed in Claim 11 wherein the surface active agent is a C₈₋₂₂ alkyl sulphate.

13. A method as claimed in Claim 12 wherein the alkyl sulphate is an alkali metal sulphate.

14. A method as claimed in Claim 13 wherein the surface active agent is sodium dodecyl sulphate.

15. A method as claimed in any one of Claims 1 to 14 wherein the surfactant is a non-ionic surfactant.

16. A method as claimed in any one of Claims 1 to 15 wherein the amount of surface active agent used is less than 5%.

17. A method as claimed in Claim 16 wherein the amount of surface active agent is less than 2%.

18. A method as claimed in any one of Claims 1 to 17 for obtaining DNA.

19. A method as claimed in any one of Claims 1 to 18 for obtaining RNA.

20. A method as claimed in Claim 19 wherein the lysis mixture includes an agent for binding or chelating RNAse cofactors to prevent RNA being degraded during lysis.

## Patentansprüche

1. Methode zum Erhalt einer Nucleinsäure aus einer Blutprobe, die folgende Schritte umfaßt:
Behandlung des Bluts in vitro mit einem hochmolekularen Kohlenhydrat, das entlang der Kohlenhydratrückgratkette negative Ladungen aufweist,
sich Absetzen lassen der roten Blutzellen und Probenahme aus der überstehenden Flüssigkeit,
Auflösen von Zellen in der Probe der überstehenden Flüssigkeit mit einer ein oberflächenaktives Mittel und ein Eiweiß aufschließendes Enzym enthaltenden Auflösungsmischung.

2. Methode nach Anspruch 1, bei der das Blut antikoaguliertes Blut ist.

3. Methode nach Anspruch 1 oder 2, bei der die Menge des dem Blut zugegebenen Kohlenhydrats weniger als 0,1 g pro ml Blut beträgt.

4. Methode nach einem der Ansprüche 1 bis 3, bei der das Kohlenhydrat eine Molmasse von 10.000-1.000.000 Dalton aufweist.

5. Methode nach einem der Ansprüche 1 bis 4, bei der es sich bei dem Kohlenhydrat um ein aus (oder im wesentlichen aus) Amylopectin bestehendes Kohlenhydrat handelt, in das negative Ladungen eingeführt worden sind.

6. Methode nach Anspruch 5, bei der die negativen Ladungen durch Hydrolyse von Hydroxyethylethergruppen verursacht werden, die in Glucoseeinheiten des Amylopectins eingeführt worden sind.

7. Methode nach Anspruch 6, bei der der Substitutionsgrad der Hydroxyethylethergruppen weniger als 2% beträgt.

8. Methode nach einem der Ansprüche 1 bis 7, bei der die Behandlung des Bluts mit dem hochmolekularen Kohlenhydrat während einer Zeitspanne derart ausgeführt wird, daß weiße Zellen in dem überstehenden Plasma verbleiben.

9. Methode nach einem der Ansprüche 1 bis 8, bei der das im Auflösungsschritt verwendete, Eiweiß aufschließende Enzym Proteinase K ist.

10. Methode nach einem der Ansprüche 1 bis 9, bei der die Menge des bei der Auflösung verwendeten, Eiweiß aufschließenden Enzyms 1-5 mg/ml beträgt.

11. Methode nach einem der Ansprüche 1 bis 10, bei der es sich bei dem oberflächenaktiven Mittel um ein anionisches Tensid handelt.

12. Methode nach Anspruch 11, bei der das oberflächenaktive Mittel ein C₈₋₂₂-Alkylsulfat ist.

13. Methode nach Anspruch 12, bei der das Alkylsulfat ein Alkalimetallsulfat ist.

14. Methode nach Anspruch 13, bei der das oberflächenaktive Mittel Natriumdodecylsulfat ist.

15. Methode nach einem der Ansprüche 1 bis 14, bei der das Tensid ein nichtionogenes Tensid ist.

16. Methode nach einem der Ansprüche 1 bis 15, bei der die Menge des verwendeten oberflächenaktiven Mittels weniger als 5% beträgt.

17. Methode nach Anspruch 16, bei der die Menge des oberflächenaktiven Mittels weniger als 2% beträgt.

18. Methode nach einem der Ansprüche 1 bis 17 zur Erzielung von DNA.

19. Methode nach einem der Ansprüche 1 bis 18 zur Erzielung von RNA.

20. Methode nach Anspruch 19, bei der die Auflösungsmischung ein Mittel zum Binden oder Chelatisieren von RNase-Cofaktoren zum Verhindern des Abbaus der RNA während der Auflösung enthält.

## Revendications

1. Procédé pour obtenir un acide nucléique à partir d'un échantillon de sang, comprenant les étapes consistant à:
traiter le sang in vitro avec un hydrate de carbone de poids moléculaire élevé possédant des charges négatives le long du squelette de l'hydrate de carbone,
laisser les globules rouges se déposer et prélever un échantillon du liquide surnageant,
lyser les globules dans ledit échantillon du produit surnageant avec un mélange de lyse contenant un agent tensioactif et une enzyme digérant les protéines.

2. Procédé selon la revendication 1, dans lequel le sang contient des anticoagulants.

3. Procédé selon la revendication 1 ou 2, dans lequel la quantité d'hydrate de carbone ajouté au sang est inférieure à 0,1 g par ml de sang.

4. Procédé selon l'une quelconque des revendications 1 à 3, dans lequel l'hydrate de carbone possède un poids moléculaire de 10.000-1.000.000 Daltons.

5. Procédé selon l'une quelconque des revendication 1 à 4, dans lequel l'hydrate de carbone est un hydrate de carbone composé de (ou sensiblement composé de) amylopectine dans laquelle ont été introduites des charges négatives.

6. Procédé selon la revendication 5, dans lequel les charges négatives sont procurées par hydrolyse de groupes d'éther hydroxyéthylique qui ont été introduits dans des unités de glucose de l'amylopectine.

7. Procédé selon la revendication 6, dans lequel le degré de substitution des groupes d'éther hydroxyéthylique est inférieur à 2%.

8. Procédé selon l'une quelconque des revendications 1 à 7, dans lequel on effectue le traitement du sang avec l'hydrate de carbone de poids moléculaire élevé pendant un laps de temps qui permet aux globules blancs de subsister dans le plasma surnageant.

9. Procédé selon l'une quelconque des revendications 1 à 8, dans lequel l'enzyme digérant les protéines utilisée dans l'étape de lyse est la protéinase K.

10. Procédé selon l'une quelconque des revendications 1 à 9, dans lequel la quantité de l'enzyme digérant les protéines utilisée dans la lyse est de 1-5 mg/ml.

11. Procédé selon l'une quelconque des revendications 1 à 10, dans lequel l'agent tensioactif est un agent tensioactif anionique.

12. Procédé selon l'une quelconque des revendications 1 à 11, dans lequel l'agent tensioactif est un sulfate d'alkyle en C₈-C₂₂.

13. Procédé selon la revendications 12, dans lequel le sulfate d'alkyle est un sulfate de métal alcalin.

14. Procédé selon la revendication 13, dans lequel l'agent tensioactif est le dodécylsulfate de sodium.

15. Procédé selon l'une quelconque des revendications 1 à 14, dans lequel l'agent tensioactif est un agent tensioactif non-ionique.

16. Procédé selon l'une quelconque des revendications 1 à 15, dans lequel la quantité de l'agent tensioactif utilisé est inférieure à 5%.

17. Procédé selon la revendications 16, dans lequel la quantité de l'agent tensioactif est inférieure à 2%.

18. Procédé selon l'une quelconque des revendications 1 à 17 pour obtenir de l'ADN.

19. Procédé selon l'une quelconque des revendications 1 à 18 pour obtenir de l'ARN.

20. Procédé selon la revendication 19, dans lequel le mélange de lyse englobe un agent pour lier ou chélater des cofacteurs d'ARNase pour empêcher l'ARN de subir une dégradation au cours de la lyse.
